# EUROPÄISCHE PATENTSCHRIFT

(11) **EP 1 800 652 B1**
(45) Veröffentlichungstag und Bekanntmachung des Hinweises auf die Patenterteilung: **02.04.2014**
(21) Anmeldenummer: 06024172.6
(22) Anmeldetag: 22.11.2006
(51) Int. Cl.: A61K 8/35, A61K 8/41, A61K 8/49, A61K 8/97, A61Q 5/06

(54) **Tönungsmittel auf Basis von Pflanzenfarbstoffen**
Hair dyeing composition with plant dyes
Composition capillaire tinctoriale à base de colorants végétaux

(30) Priorität: 21.12.2005 DE 102005061727
(43) Veröffentlichungstag der Anmeldung: 27.06.2007
(73) Patentinhaber: Henkel AG & Co. KGaA, 40589 Düsseldorf (DE)
(72) Erfinder: Kleen, Astrid, 22763 Hamburg (DE); Terrier, Janie, 22589 Hamburg (DE)

(56) Entgegenhaltungen:
- EP-A1- 0 630 643
- EP-A1- 0 801 942
- DE-C1- 3 829 102
- DE-C1- 19 713 696
- DE-U1- 20 011 207
- MAUREEN JONES: "Healthy Hair With Henna" BODY ART CENTRAL NEWSLETTER, [Online] Nr. 2, April 2005 (2005-04), XP002429098 Gefunden im Internet: URL:http://www.everydayhenna.com/newslette r/aprilnewsletter.pdf> [gefunden am 2007-04-12]
- A. BEHLER AND AL: "Quaternäre Ammonium-Verbindungen", RÖMPP ONLINE, VERSION 3.10, 2010, Retrieved from the Internet: URL:http://www.roempp.com> [retrieved on 2010-11-30]
- ANONYM: "Quartäre Ammoniumverbindungen", WIKIPEDIA, DIE FREIE ENZYKLOPÄDIE, 31 October 2010 (2010-10-31), Retrieved from the Internet: URL:http://de.wikipedia.org/wiki/Quart%C3% A4re_Ammoniumverbindungen> [retrieved on 2010-11-30]
- "quarernary ammonium salt" In: R. J. Lewis: "Hawley's Condensed Chemical Dictionary, 13th edition", 1997, John Wiley & Sons, INC., New York ISBN: 0-471-29205-2 page 949,

## Beschreibung

Gegenstand der vorliegenden Erfindung ist ein Verfahren zur Tönung keratinischer Fasern, bei dem die Fasern mit mindestens einem Naturfarbstoff und mindestens einem synthetischen kationischen Farbstoff behandelt werden.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Haar aufziehen und keinen oxidativen Prozess zur Ausbildung der Farbe benötigen. Diese Färbungen sind gegen Shampoonieren in der Regel deutlich empfindlicher als die oxidativen Färbungen, so dass sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Dennoch weist diese neuartige Methode den Nachteil auf, dass zwar natürliche Nuancen gut erzielt werden können, aber modische Farbtöne, beispielsweise im Rotbereich nicht zur Verfügung stehen.

Trotz dieser Vielfalt an modernen Haarfärbemethoden erfreuen sich die Pflanzenfarben auf Basis von Naturfarbstoffen, wie beispielsweise Henna, weiterhin einer hohen Beliebtheit. Allerdings weisen diese Färbemittel häufig ein schlechtes Aufziehverhalten, wenig überzeugende Echtheitseigenschaften sowie eine geringe Grauabdeckung auf.

Aufgabe der vorliegenden Erfindung war es daher, ein Verfahren zur Färbung keratinischer Fasern auf Basis von Naturfarbstoffen zu entwickeln, dass die oben genannten Nachteile nicht aufweist.

Es wurde nunmehr überraschend gefunden, dass ein Tönungsverfahren, bei dem die Fasern mit einem Naturfarbstoff und einem synthetischen kationischen direktziehenden Farbstoff behandelt werden, Tönungen mit einer deutlich verbesserten Haltbarkeit und Pflegeleistung ermöglichen.

Ein erster Gegenstand der vorliegenden Erfindung ist daher ein Verfahren zur Tönung keratinischer Fasern, bei dem die Fasern mit mindestens einem Naturfarbstoff und mindestens einem synthetischen kationischen direktziehenden Farbstoff behandelt werden, dadurch gekennzeichnet, dass die Fasern
- in einem ersten Schritt mit einer Zubereitung (A), enthaltend mindestens einen Naturfarbstoff, behandelt werden,
- anschließend ggf. gespült werden und
- in einem zweiten Schritt mit einer Zubereitung (B), enthaltend mindestens einen synthetischen kationischen direktziehenden Farbstoff, der mindestens einen Heterocyclus aufweist, der mindestens ein quaternäres Stickstoffatom enthält, behandelt werden.

Unter keratinischen Fasern sind dabei erfindungsgemäß Pelze, Wolle, Federn und insbesondere menschliche Haare zu verstehen.

Im Rahmen der vorliegenden Erfindung sind verschiedene Ausführungsformen des Verfahrens erfindungsgemäß bevorzugt.

Die im Rahmen des erfindungsgemäßen Verfahrens eingesetzten Zubereitungen (A) und (B) enthalten die Farbstoffe bevorzugt in einem geeigneten wässrigen, alkoholischen oder wässrig-alkoholischen Träger. Zum Zwecke der Haartönung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Lösungen sind im Sinne der vorliegenden Erfindung wässrige Lösungen enthaltend 3 bis 70 Gew.% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Weiterhin hat es sich als erfindungsgemäß bevorzugt erwiesen, wenn die Anwendungszubereitung, enthaltend den Naturfarbstoff, unmittelbar vor der Anwendung aus einer pulverförmigen Zubereitung, enthaltend mindestens einen Naturfarbstoff und gegebenenfalls mindestens einen synthetischen kationischen direktziehenden Farbstoff, und einer wässrigen Zubereitung (C) hergestellt wird.

Dazu hat es sich als vorteilhaft erwiesen, wenn das pulverförmige Produkt, enthaltend mindestens einen Naturfarbstoff, im Verhältnis 1 : 1 bis 1 : 10, vorzugsweise von 1 : 2 bis 1 : 4, mit einer wässrigen Zubereitung, vorzugsweise mit einer heißen wässrigen Zubereitung, ganz bevorzugt mit heißem Wasser gemischt wird.

Obwohl prinzipiell eine Anwendung auf feuchten Fasern denkbar ist, ist es erfindungsgemäß bevorzugt, wenn die Zubereitung (A) auf trockene Fasern aufgetragen wird.

Obwohl ein Spülschritt zwischen den beiden tönenden Behandlungen erfindungsgemäß nicht erforderlich ist, hat es sich im Rahmen der vorliegenden Erfindung als bevorzugt erwiesen, wenn die Fasern zwischen der Anwendung der beiden farbverändernden Mittel gründlich gespült und gegebenenfalls shampooniert werden.

Die Zubereitung (B) wird dann bevorzugt auf die handtuchtrockenen Fasern aufgetragen.

Im Rahmen dieser Ausführungsform ist es bevorzugt, wenn die Einwirkzeit E1 der Zubereitung (A) zwischen 10 und 90 Minuten, vorzugsweise zwischen 30 und 70 Minuten und insbesondere 30 bis 70 Minuten beträgt.

Im Rahmen dieser Ausführungsform ist es ferner bevorzugt, wenn die Einwirkzeit E2 der Zubereitung (B) zwischen 1 und 40 Minuten, vorzugsweise zwischen 5 und 25 Minuten und insbesondere 15 bis 20 Minuten beträgt.

Obwohl jede der Zubereitungen (A) oder (B) jeden der beiden Farbstofftypen enthalten kann, enthält die Zubereitung (A) mindestens einen Naturfarbstoff und die Zubereitung (B) mindestens einen synthetischen kationischen direktziehenden Farbstoff, der mindestens einen synthetischen kationischen direktziehenden Farbstoff, der mindestens einen Heterocyclus aufweist, der mindestens ein quaternäres Stickstoffatom enthält.

Als Naturfarbstoffe eignen sich dabei alle in Pflanzen, Tieren und Mikroorganismen enthaltenen und aus diesen gewinnbaren Farbstoffe, insbesondere solche, die bereits zur Färbung von Keratinfasern oder von Haaren verwendet worden sind. Eine Übersicht über natürliche Farbstoffe ist aus Ullmanns Encyclopädie der technischen Chemie, 4. Auflage (19), Band 11, Seite 100 - 134 zu entnehmen.

Im Rahmen der vorliegenden Erfindung hat es sich als vorteilhaft erwiesen, wenn der Naturfarbstoff ausgewählt ist aus Henna rot, Henna neutral, Henna schwarz, Kamillenblüte, Sandelholz, schwarzem Tee, grünem Tee, Faulbaumrinde, Salbei, Blauholz, Krappwurzel, Catechu, Sedre, Alkannawurzel, Walnussschale, Indigo und Cochenille.

Die im Rahmen des erfindungsgemäßen Verfahrens eingesetzten Mittel enthalten die Naturfarbstoffe bevorzugt in einer Menge von 0,01 bis 20 Gew.%, bezogen auf die gesamte Anwendungszubereitung.

Erfindungsgemäß bevorzugt sind die Verbindungen, die mindestens einen Heterocyclus aufweisen, der mindestens ein quaternäres Stickstoffatom enthält, ausgewählt ist aus den Verbindungen der Formeln (DZ1) bis (DZ7)

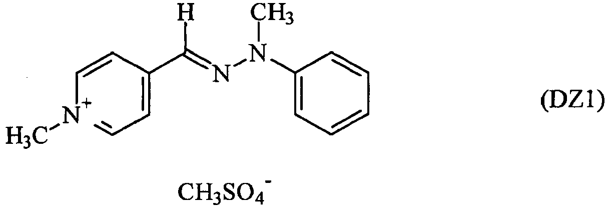

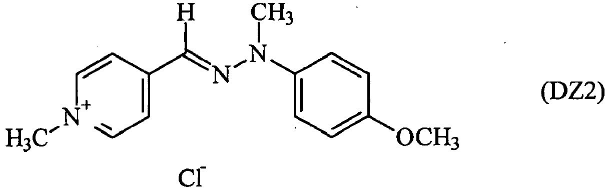

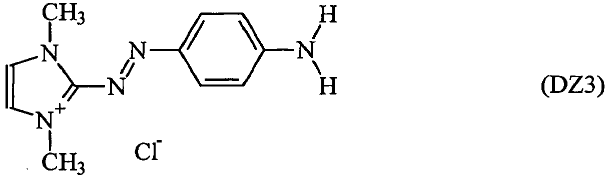

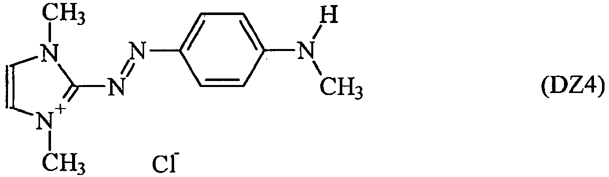

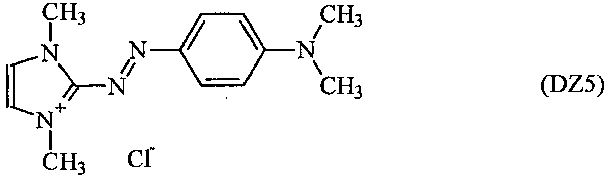

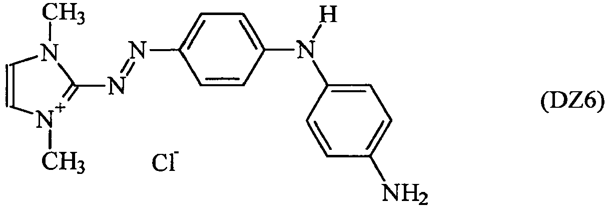

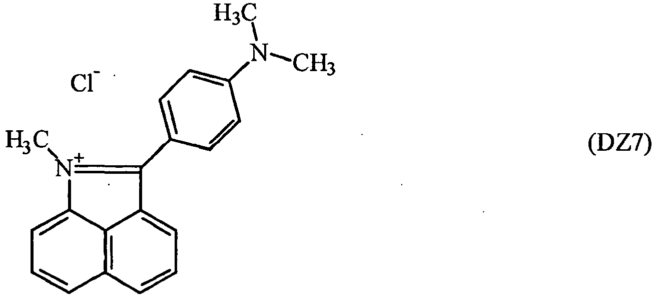

Die im Rahmen des erfindungsgemäßen Verfahrens eingesetzten Mittel enthalten die kationischen synthetischen direktziehenden Farbstoffe bevorzugt in einer Menge von 0,01 bis 20 Gew.-%, bezogen auf die gesamte Anwendungszubereitung.

Neben den erfindungswesentlichen Farbstoffen können die im Rahmen des erfindungsgemäßen Verfahrens eingesetzten Mittel einen weiteren synthetischen direktziehenden Farbstoff enthalten. Die direktziehenden Farbstoffe sind vorzugsweise ausgewählt aus den Nitrophenylendiaminen, den Nitroaminophenolen, den Azofarbstoffen, den Anthrachinonen oder den Indophenolen.

Besonders bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Die im Rahmen des erfindungsgemäßen Verfahrens eingesetzten Mittel enthalten die weiteren direktziehenden Farbstoffe bevorzugt in einer Menge von 0,01 bis 20 Gew.%, bezogen auf die gesamte Anwendungszubereitung.

Es ist nicht erforderlich, dass die direktziehenden Farbstoffe jeweils einheitliche Verbindungen darstellen. Vielmehr können in den erfindungsgemäßen Haartönungsmitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haartönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Im Rahmen einer bevorzugten Ausführungsform der vorliegenden Erfindung kann es besonders bevorzugt sein, wenn mindestens eine der Zubereitungen (A) und/oder gegebenenfalls (B) und/oder gegebenenfalls (C) mindestens einen Pflegestoff enthält.

Dabei kann es bevorzugt sein, wenn der Pflegestoff natürlich, natürlichen Ursprungs oder zumindest naturidentisch ist. Besonders bevorzugt sind erfindungsgemäß Pflegestoffe ausgewählt ist aus Vitaminen, Proteinhydrolysaten und Pflanzenextrakten.

In einer Variante der vorliegenden Erfindung enthält mindestens eine der Zubereitungen (A) und/oder gegebenenfalls (B) und/oder gegebenenfalls (C) mindestens eine Vitamin, ein Pro-Vitamin oder eine Vitaminvorstufe. Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung, enthalten. Mengen von 0,1-5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.%, bezogen auf die gesamte Anwendungszubereitung, enthalten. Mengen von 0,05 -1 1 Gew.-% sind besonders bevorzugt.

Vitamin C (Ascorbinsäure). Vitamin C wird in den erfindungsgemäß verwendeten Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.%, insbesondere in Mengen von 0,001 bis 0,01 Gew.- %, jeweils bezogen auf die gesamte Anwendungszubereitung enthalten.

Bevorzugt enthalten die erfindungsgemäßen Zweikomponentenmittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

In einer weiteren Variante der vorliegenden Erfindung enthält mindestens eine der Zubereitungen (A) und/oder gegebenenfalls (B) und/oder gegebenenfalls (C) mindestens ein Proteinhydrolysat und/oder eine Aminosäure.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid.

Selbstverständlich können erfindungsgemäß auch ß-Aminosäuren und deren Derivate wie ß-Alanin, Anthranilsäure oder Hippursäure eingesetzt werden. Erfindungsgemäß bevorzugte Aminosäuren sind Serin, Glycin und Lysin.

Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in der erfindungsgemäßen Zubereitung in Konzentrationen von 0,01 Gew. % bis zu 20 Gew. %, vorzugsweise von 0,05 Gew. % bis zu 15 Gew. % und ganz besonders bevorzugt in Mengen von 0,05 Gew. % bis zu 5 Gew. % enthalten.

In einer weiteren Variante der vorliegenden Erfindung enthält mindestens eine der Zubereitungen (A) und/oder gegebenenfalls (B) und/oder gegebenenfalls (C) mindestens einen Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Die im Rahmen des erfindungsgemäßen Verfahrens eingesetzten Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylehglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾. oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die im Rahmen des erfindungsgemäßen Verfahrens eingesetzten Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte G12-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, VinylpyrrolidonlVinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die folgenden Beispiele sollen den Gegenstand der vorliegenden Anmeldung näher erläutern ohne diesen in irgendeiner Weise zu beschränken.

### Beispiele:

Die Mengenangaben im Rahmen der folgenden Rezepturen verstehen sich, sofern nichts anderes vermerkt ist in Gewichtsprozent.

Es wurden die folgenden Rezepturen hergestellt:
*1 Zubereitung, enthaltend Naturfarbstoff:*

| Rohstoffe | Menge |
|---|---|
| Henna rot | 2,0 |
| Walnussschalen | 48,0 |
| Henna neutral | 50,0 |

*2 Zubereitung mit kationischen Direktziehem:*

| Rohstoff | Menge |
|---|---|
| Methylparaben | 0,2 |
| Genapoll^{®} LRO flüssig | 7,8 |
| Tetrasodium EDTA | 0,2 |
| Ammoniumcarbopollösung | 35,0 |
| Lowenol^{®} C-279 | 0,4 |
| Polawax^{®} GP 200 | 1,5 |
| Cetylalkohol | 1,7 |
| Lanette^{®} 14 | 1,2 |
| Pionier^{®} 6508 | 4,0 |
| Emulgade^{®} 1000 | 3,5 |
| Basic Yellow 87 | 0,1 |
| Basic Orange 31 | 0,1 |
| Polyquaternium-6 | 0,5 |
| Parfum | 0,3 |
| 2-Amino-2-methylpropanol | 0,2 |
| Wasser | ad 100 |

*3 Anwendung*
Die Haare der Probanden wurden entlang des Scheitels in zwei Hälften geteilt. Auf die eine Hälfte (trockenes Haar) wurde zunächst eine Pflanzenhaarfarbe angewendet. Dazu wurde die Formulierung (siehe Punkt 1) im Verhältnis 1:3 bis 1:4 mit heißem Wasser gemischt und vom Haaransatz bis zu den Spitzen aufgetragen. Die Einwirkzeit betrug 40 Minuten unter Wärmezufuhr.
Dann wurde das gesamte Haar der Probanden gespült und shampooniert, solange bis Haar und Haut völlig sauber waren.
Dann wurde auf das gesamte handtuchtrockene Haar die Zubereitung, enthaltend mindestens einen kationischen direktziehenden Farbstoff (Punkt 2), appliziert. Die Einwirkzeit betrug 25 Minuten. Abschließend wurden die Haare gespült und sanft shampooniert.
*4 Ergebnis*
Es wurde die Farbabtragung nach 6 Wäschen und die Pflegewirkung von insgesamt 5 geschulten Personen beurteilt. Dabei wurden die folgenden Ergebnisse erhalten:

| | Vergleich | Erfindung |
|---|---|---|
| | Behandlung nur mit kationischen Direktzieher | Naturfarbstoff und kationischer Direktzieher |
| Farbabstand nach 6 Wäschen | ΔE = 11,06 | ΔE = 8,90 |
| Pflegewirkung | + | +++ |

Zur Berechnung des Farbabstandes nach 6 Wäschen wurden die Fasern unmittelbar nach dem Tönungsvorgang sowie nach anschließenden 6 Wäschen farbmetrisch nach dem L,a,b-System vermessen.
Die Ergebnisse zeigen deutlich, dass die erfindungsgemäß behandelten Haare einen deutlich besseren Pflegezustand aufwiesen, als die Haare, die mit einem Mittel des Standes der Technik behandelt worden waren.
Ferner kann an den ΔE-Werten abgelesen werden, dass die resultierende Färbung nach 6 Haarwäschen im Fall des erfindungsgemäßen Verfahrens weniger stark von der Färbung unmittelbar nach der Tönung abweicht (kleinerer ΔE-Wert) und somit die erfindungsgemäße Färbung eine bessere Waschechtheit aufweist.
*5 Verzeichnis der eingesetzten Handelsprodukte*

| | |
|---|---|
| Ammoniumcarbopollsg. | 1%ige Lösung eines Ammoniumsalzes eines Methacrylsäure-Methylacrylat-Copolymeren (INCI-Bezeichnung: Ammonium Polyacrylate) (Röhm GmbH) |
| Emulgade^{®} 1000 | INCI-Bezeichnung: Cetearyl Alcohol, Ceteareth-20 (Cognis) |
| Genapol^{®} LRO flüssig | Natriumlaurylethersulfat (ca. 27% Aktivsubstanzgehalt; INCI-Bezeichnung: Sodium Laureth Sulfate) (Clariant) |
| Lanette^{®} 14 | Myristylalkohol (INCI-Bezeichnung: Myristyl Alcohol) (Cognis) |
| Lowenol^{®} C-279 | Fettalkohol mit ca. 2 EO-Einheiten (mindestens 99% Aktivsubstanzgehalt; INCI-Bezeichnung: Ceteareth-2) (Lowenstein) |
| Pionier^{®} 6508 | Paraffinöl (INCI-Bezeichnung: Mineral (Paraffinum Liquidum) Oil) (Hansen & Rosenthal) |
| Polawax^{®} GP 200 | INCI-Bezeichnung: Cetearyl Alcohol, PEG-20 Stearate (Croda) |

## Patentansprüche

1. Verfahren zur Tönung keratinischer Fasern, bei dem die Fasern mit mindestens einem Naturfarbstoff und mindestens einem synthetischen kationischen direktziehenden Farbstoff behandelt werden, **dadurch gekennzeichnet, dass** die Fasern
- in einem ersten Schritt mit einer Zubereitung (A), enthaltend mindestens einen Naturfarbstoff, behandelt werden,
- anschließend ggf. gespült werden und
- in einem zweiten Schritt mit einer Zubereitung (B), enthaltend mindestens einen synthetischen kationischen direktziehenden Farbstoff, der mindestens einen Heterocyclus aufweist, der mindestens ein quaternäres Stickstoffatom enthält, behandelt werden.

2. Verfahren nach Anspruch 1, **dadurch gekennzeichnet, dass** der Naturfarbstoff ausgewählt ist aus Henna rot, Henna neutral, Henna schwarz, Kamillenblüte, Sandelholz, schwarzem Tee, grünem Tee, Faulbaumrinde, Salbei, Blauholz, Krappwurzel, Catechu, Sedre, Alkannawurzel, Walnussschale, Indigo und Cochenille.

3. Verfahren nach einem der Ansprüche 1 oder 2, **dadurch gekennzeichnet, dass** die Verbindung, die mindestens einen Heterocyclus aufweist, der mindestens ein quaternäres Stickstoffatom enthält, ausgewählt ist aus den Verbindungen der Formeln (DZ1) bis (DZ7)

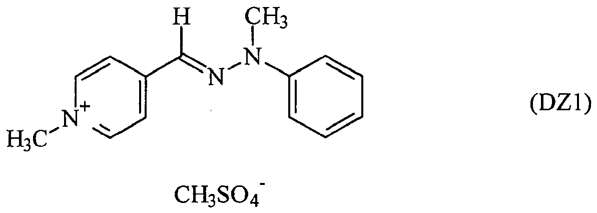

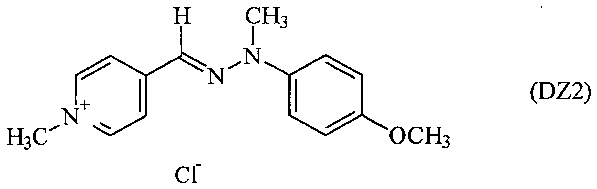

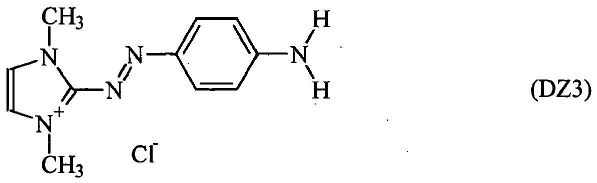

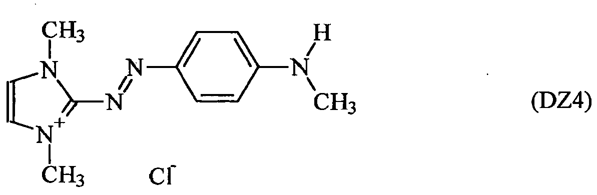

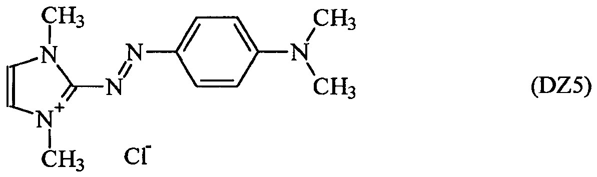

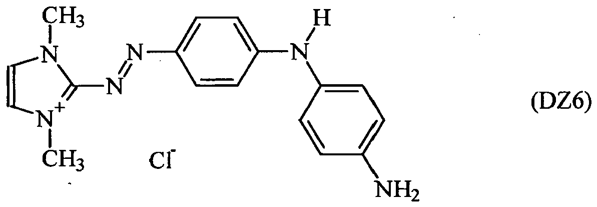

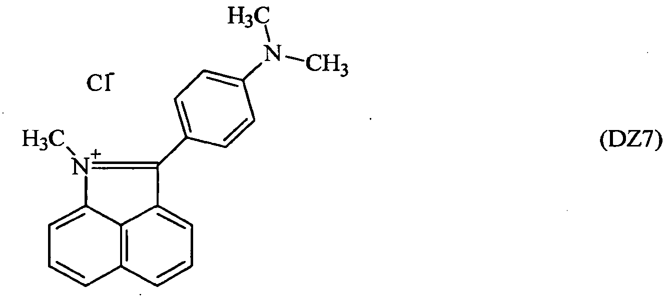

4. Verfahren nach einem der Ansprüche 2 oder 3, **dadurch gekennzeichnet, dass** die Zubereitung (A) unmittelbar vor der Anwendung aus einer pulverförmigen Zubereitung, enthaltend mindestens einen Naturfarbstoff und gegebenenfalls mindestens einen synthetischen kationischen direktziehenden Farbstoff, der mindestens einen Heterocyclus aufweist, der mindestens ein quaternäres Stickstoffatom enthält, und einer wässrigen Zubereitung (C) hergestellt wird.

5. Verfahren nach einem der Ansprüche 1 bis 4, **dadurch gekennzeichnet, dass** mindestens eins der eingesetzten Mittel mindestens einen Pflegestoff enthält.

6. Verfahren nach Anspruch 5, **dadurch gekennzeichnet, dass** der Pflegestoff natürlichen Ursprungs ist.

7. Verfahren nach Anspruch 6, **dadurch gekennzeichnet, dass** der Pflegestoff ausgewählt ist aus Vitaminen, Aminosäuren und Pflanzenextrakten.

## Claims

1. A method for tinting keratinic fibres in which the fibres are treated with at least one natural dye and at least one synthetic cationic substantive dye, **characterised in that** the fibres
- are treated in a first step with a preparation (A) containing at least one natural dye,
- are then optionally rinsed and
- are treated in a second step with a preparation (B) containing at least one synthetic cationic substantive dye which comprises at least one heterocycle which contains at least one quaternary nitrogen atom.

2. A method according to claim 1, **characterised in that** the natural dye is selected from henna red, henna neutral, henna black, chamomile flowers, sandalwood, black tea, green tea, alder buckthorn bark, sage, logwood, madder root, catechu, lotus tree, alkanet root, walnut shell, indigo and cochineal.

3. A method according to either of claim 1 or claim 2, **characterised in that** the compound which comprises at least one heterocycle which contains at least one quaternary nitrogen atom is selected from the compounds of the formulae (DZ1) to (DZ7)

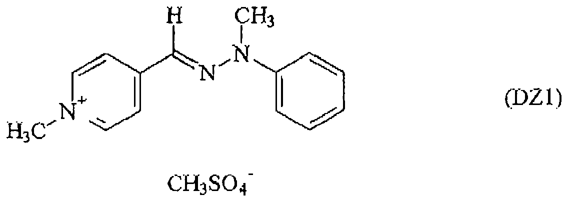

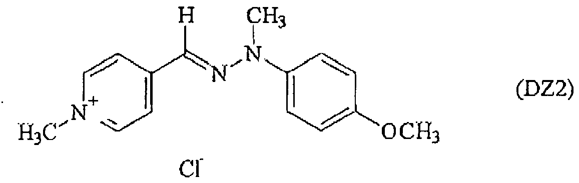

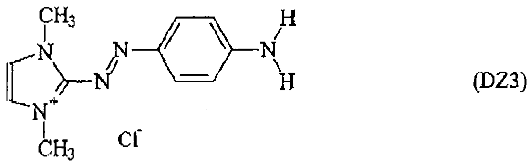

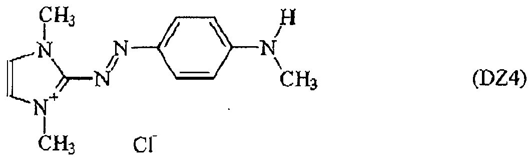

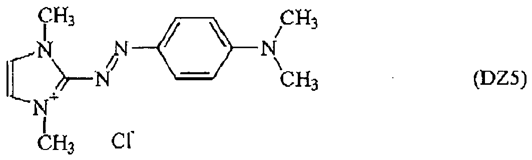

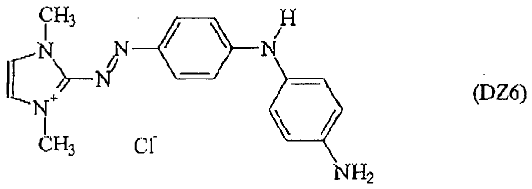

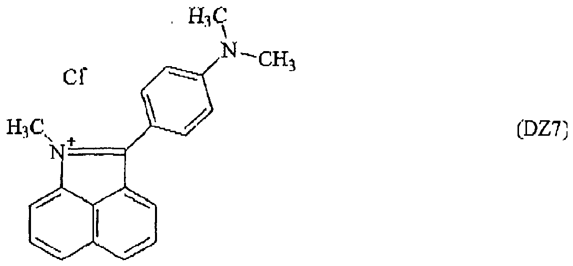

4. A method according either of claim 2 or claim 3, **characterised in that** the preparation (A) is produced immediately before use from a pulverulent preparation containing at least one natural dye and optionally at least one synthetic cationic substantive dye which comprises at least one heterocycle which contains at least one quaternary nitrogen atom, and an aqueous preparation (C).

5. A method according to any one of claims 1 to 4, **characterised in that** at least one of the agents used contains at least one conditioner.

6. A method according to claim 5, **characterised in that** the conditioner is of natural origin.

7. A method according to claim 6, **characterised in that** the conditioner is selected from vitamins, amino acids and plant extracts.

## Revendications

1. Procédé pour la teinture de fibres kératiniques, dans lequel on traite les fibres avec au moins un colorant naturel et au moins un colorant synthétique cationique direct, **caractérisé en ce qu'**on traite les fibres
- dans une première étape avec une préparation (A) contenant au moins un colorant naturel ;
- ensuite on les rince de manière facultative ; et
- dans une deuxième étape, on les traite avec une préparation (B) contenant au moins un colorant synthétique cationique direct qui présente au moins un hétérocycle qui contient au moins un atome d'azote quaternaire.

2. Procédé selon la revendication 1, **caractérisé en ce que** le colorant naturel est choisi parmi le henné rouge, le henné neutre, le henné noir, la fleur de camomille, le bois de santal, le thé noir, le thé vert, l'écorce de bourdaine, la sauge, le bois de campêche, la racine de garance, le cachou, le cèdre, la racine d'alkanna, la coquille de noix, l'indigo et la cochenille.

3. Procédé selon l'une quelconque des revendications 1 ou 2, **caractérisé en ce que**, le composé qui présente au moins un hétérocycle qui contient au moins un atome d'azote quaternaire est choisi parmi les composés répondant aux formules (DZ1) à (DZ7) :

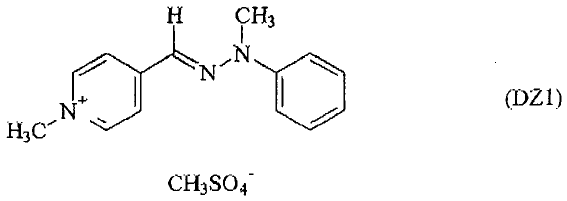

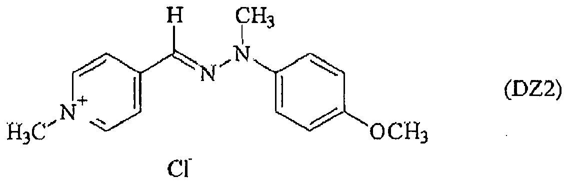

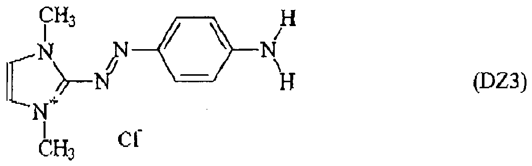

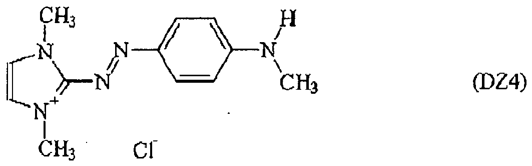

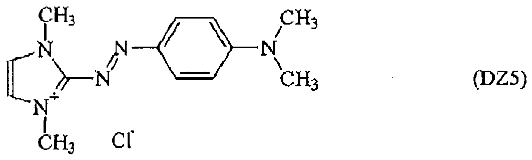

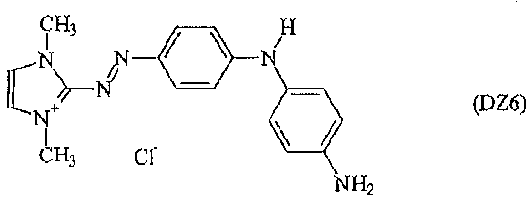

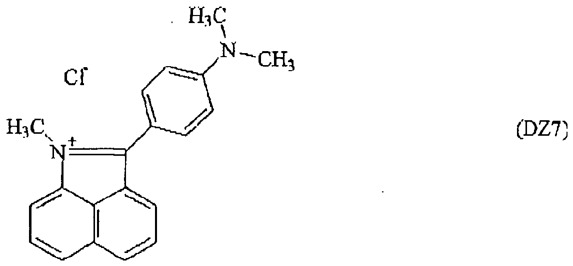

4. Procédé selon l'une quelconque des revendications 2 ou 3, **caractérisé en ce qu'**on prépare la préparation (A) directement avant l'emploi à partir d'une préparation pulvérulente contenant au moins un colorant naturel et de manière facultative au moins un colorant synthétique cationique direct qui présente au moins un hétérocycle qui contient au moins un atome d'azote quaternaire, et à partir d'une préparation aqueuse (C).

5. Procédé selon l'une quelconque des revendications 1 à 4, **caractérisé en ce qu'**au moins un des agents mis en oeuvre contient au moins une substance d'entretien.

6. Procédé selon la revendication 5, **caractérisé en ce que** la substance d'entretien est d'origine naturelle.

7. Procédé selon la revendication 6, **caractérisé en ce que** la substance d'entretien est choisie parmi des vitamines, des acides aminés et des extraits de plantes.
